# EUROPÄISCHE PATENTSCHRIFT

(11) **EP 0 553 792 B1**
(45) Veröffentlichungstag und Bekanntmachung des Hinweises auf die Patenterteilung: **26.05.1999**
(21) Anmeldenummer: 93101216.5
(22) Anmeldetag: 27.01.1993
(51) Int. Cl.: C07D 501/36, A61K 31/545, C07D 501/18

(54) **Verfahren zur Herstellung von Ceftriaxon-dinatriumsalzhemiheptahydrat**
Process for the preparation of the disodium salt hemiheptahydrate of ceftriaxone
Procédé pour la préparation de l'hémiheptahydrate du sel disodé de ceftriaxone

(30) Priorität: 28.01.1992 AT 132/92
(43) Veröffentlichungstag der Anmeldung: 04.08.1993
(73) Patentinhaber: LEK, tovarna farmacevtskih in kemicnih izdelkov, d.d., Ljubljana, SI-61107 Ljubljana (SI)
(72) Erfinder: Hafner-Milac, Natasa, Dipl.-Ing., 61000 Ljubjana (SL); Langof, Igor, Dipl.-Ing., 61000 Ljubjana (SL); Rusjakovski, Boris, Dipl.-Ing., 61000 Ljubjana (SL); Borisek, Sandi, 61000 Ljubljana (SL); Jereb, Darja, 61235 Rodomlje (SL)
(74) Vertreter: Müller-Boré & Partner Patentanwälte

(56) Entgegenhaltungen:
- EP-A- 0 005 830
- EP-A- 0 030 294
- EP-A- 0 037 380
- EP-A- 0 175 814
- EP-A- 0 399 094
- DE-A- 3 720 681
- CHEMICAL ABSTRACTS, vol. 99, no. 9, 29. August 1983, Columbus, Ohio, US; abstract no. 70458y, Seite 602 ;Spalte R ; & ES-A-510 270 (SERVICIOS Y SUMINISTROS FARMACEUTICOS)

## Beschreibung

Die Erfindung betrifft ein neues und verbessertes Verfahren zur Herstellung von Ceftriaxondinatriumsalz-hemiheptahydrat der Formel I 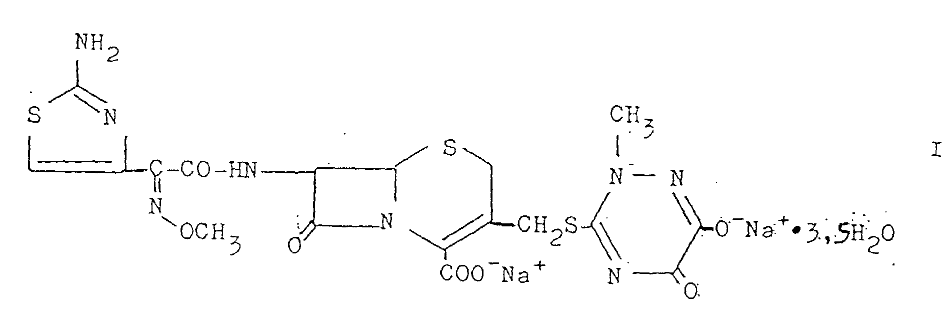 Ceftriaxon ist die generische Bezeichnung für die 7-{[2-(2-Aminothiazol-4-yl)-2-*syn*-methoxyimino]acetamido}-3-{[(2,5-dihydro-6-hydroxy-2-methyl-5-oxo-*as*-triazin-3-yl)thio]methyl}-3-cephem-4-carbonsäure. Die Verbindung ist ein halbsynthetisches Cephalosporin-Antibiotikum dritter Generation und wird in der Form des Dinatriumsalz-hemiheptahydrats zur parenteralen Anwendung bei der Behandlung von schweren Infektionen, wie akuten und chronischen Infektionen des Urinartraktes, von respiratorischen Infektionen, Endocarditis, Meningitis u.dgl. verwendet.

Die Erfindung betrifft weiterhin 7-Amino-3-{[2,5-dihydro-6-hydroxy-2-methyl-5-oxo-as-triazin-3-yl)thio]-methyl}-3-cephem-4-carbonsäure-dinatriumsalz mit der folgenden Formel II:

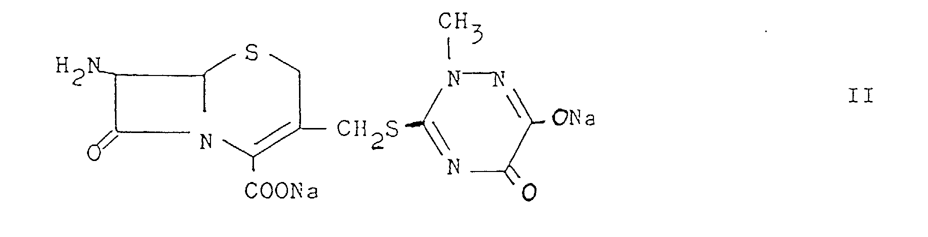

Dieses neue Zwischenprodukt wird durch quantitative Neutralisation der 7-Amino-3-{[2,5-dihydro-6-hydroxy-2-methyl-5-oxo-as-triazin-3-yl)thio]methyl}-3-cephem-4-carbonsäure in Form der freien Säure mit einer wäßrigen NaHCO₃-Lösung hergestellt.

In EP-B-0005830 werden neue Derivate der 7-[2-(2-Amino-4-thiazolyl)-2-(methoxyimino)-acetamido]-cephalosporansäure der allgemeinen Formel 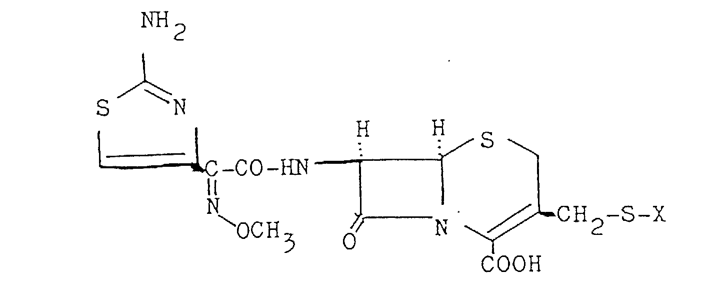 worin X eine 2,5-Dihydro-6-hydroxy-2-methyl-5-oxo-*as*-triazin-3-yl-gruppe bzw. deren entsprechende tautomere Form, die 1,2,5,6-Tetrahydro-2-methyl-5,6-dioxo-*as*-triazin-3-yl-gruppe gemäss der folgenden Struktur 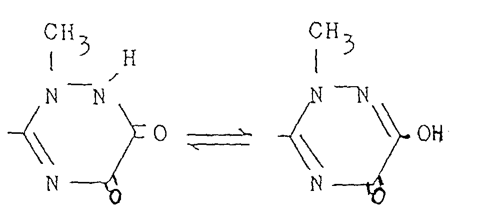 bedeutet, sowie Ester/Ether und Salze dieser Verbindung und deren Hydrate beschrieben und beansprucht werden, wobei die Verbindungen der obigen Formel in der syn-isomeren oder in der anti-isomeren Form bzw. als Gemische dieser beiden Formen vorliegen.

Als bevorzugte Produkte werden die (6R,7R)-7-[2-(2-Amino-4-thiazolyl)-2-(Z-methoxyimino)acetamido]-3-{[(2,5-dihydro-6-hydroxy-2-methyl-5-oxo-*as*-triazin-3-yl)thio]-methyl)-8-oxo-5-thia-1-azabicyclo[4.2.0]oct-2-en-2-carbonsäure und deren Salze sowie die entsprechenden Hydrate bzw. Hydrate der Salze in der EP-B-5830 beansprucht.

Diese Verbindung ist jetzt unter der generischen Bezeichnung Ceftriaxon bekannt. Ceftriaxon ist ein wertvolles Cefalosporin-Antibiotikum mit breitem Wirkungsspektrum gegen Gram-positive sowie Gram-negative Mikroorganismen einschliesslich β-Laktamase bildender Staphylokokken und verschiedener β-Laktamase bildender Gram-negativer Bakterien wie z.B. *Pseudomonas aeruginosa, Haemophilus influenzae, Escherichia coli, Serratia marascens, Proteus*- und *Klebsiella-Gattungen*.

Das Herstellungsverfahren beruht auf bekannten Methoden zur Herstellung von strukturähnlichen Verbindungen, beschrieben in der DE-A-2527291 bzw. in der äquivalenten US-A-4 091 211.

Die Synthese von Ceftriaxon gemäss dem in EP-B-0005830 beschriebenen Verfahren beruht auf der Abspaltung der Schutzgruppe aus dessen Precursor, d.h. aus der Verbindung der Formel 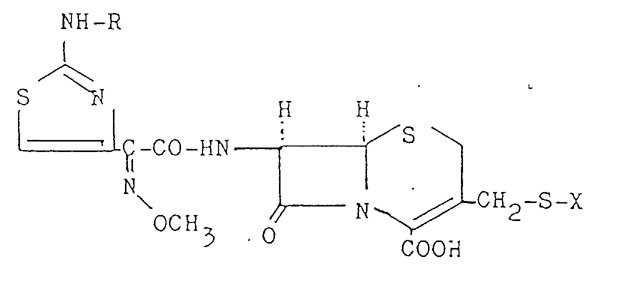 worin X die obengenannte Bedeutung hat (keto-enolische Tautomerie), R eine leicht abspaltbare Schutzgruppe wie eine Trityl- oder Chloracetyl-gruppe bedeutet und Carboxygruppe ggf. auch geschützt werden kann, und anschliessender Umsetzung von Ceftriaxon zu dessen Salzen oder Hydraten bzw. Hydraten von dessen Salzen.

Es können Mono- oder Disalze des Ceftriaxons hergestellt werden, wobei die Bildung des Disalzes an der Hydroxygruppe der Enolform, d.h. der 2,5-Dihydro-6-hydroxy-2-methyl-5-oxo-*as*-triazin-3-yl-gruppe, erfolgen kann.

Es wird die Möglichkeit der Herstellung von Alkali- und Erdalkalisalzen sowie Salzen mit organischen Basen wie Mono- und Diaminen beschrieben. Die Herstellung der Hydrate erfolgt zumeist automatisch im Zuge des Herstellungsverfahrens oder als Folge der hygroskopischen Eigenschaften des zunächst wasserfreien Produkts.

Für parenterale Zubereitungen kann das Lyophilisat des Ceftriaxon-dinatrumsalzes hergestellt werden, das in Ampullen abgefüllt wird. Vor Gebrauch wird das Lyophilisat mit einer 2%-igen wäßrigen Lidocainhydrochlorid-Lösung versetzt.

Gemäss dem in EP-B-0005830 beschriebenen Verfahren kann das erhaltene *syn/anti*-Gemisch der gewünschten Verbindung auf übliche Weise wie durch Umkristallisation oder durch chromatographische Methoden unter Verwendung eines geeigneten Lösungsmittels bzw. Lösungsmittelgemisches aufgetrennt werden.

In der einschlägigen Literatur sind noch einige verbesserte Verfahren zur Synthese von Ceftriaxon (als Carbonsäure) beschrieben, die auf der Auswahl von neuen und verbesserten Acylierungsmitteln, d.h. 2-(2-Aminothiazol-4-yl)-2-*syn*-methoxyiminoessigsäure-Derivaten, die in der *syn*-isomeren Form mit grosser Reinheit vorliegen, wobei die *syn*-Konfiguration in allen Stufen der Umsetzung, auch in der Acylierungsstufe erhalten bleibt, beruhen.

Bei den vorbekannten reaktionsfähigen Derivaten, besonders bei den aktivierten Estern, kann es während der Umsetzung wegen der einigermassen unstabilen *syn*-Konfiguration zu einem erhöhten Anteil an *anti*-Isomeren und dadurch zu einer geringeren Ausbeute am gewünschen *syn*-Isomer kommen.

Bei Verwendung verbesserter Acylierungsmittel mit *syn*-Konfiguration bleibt diese bei der Umsetzung erhalten und die gewünschte Substanz (Ceftriaxon) wird in grosser Reinheit und Ausbeute erhalten; darüberhinaus besteht kein Bedürfnis nach dem Schutz des Amino-Substituenten im Tiazolylring der Seitenkette, wie in der EP-A-0175814, DE-A-37 20 681 oder in EP-A-0037380 bzw. ihrem Äquivalent US-A-4 767 852 beschrieben ist. Gemäss dem letztgenannten Verfahren wird die 7-Amino-3-(2,5-dihydro-2-methyl-6-hydroxy-5-oxo-*as*-triazin-3-yl)thiomethyl-3-cephem-4-carbonsäure, worin die Carboxygruppe durch eine Trimethylsilylgruppe geschützt ist, mit dem neuen aktivierten Ester, d.h. mit dem 2-(2-Aminothiazol-4-yl)-2-*syn*-methoxyiminoessigsäure-2-benzthiazolyl-thioester, acyliert.

Durch den Schutz der Carboxygruppe mit dem Silylierungsmittel wird die Löslichkeit der an sich sehr schwer löslichen Ausgangskomponente erhöht und ausserdem erfolgt wegen der hygroskopischen Eigenschaften der Silylierungsverbindung die Acylierung in einem wasserfreien Medium und in einer inerten Atmosphäre.

Nach erfolgter Acylierung und Isolation des Ceftriaxons in reiner Form und hoher Ausbeute (85%) konnte das Ceftriaxon-dinatriumsalz durch Umsetzung des Ceftriaxons (in Form von freier Säure) mit Natrium-2-ethylhexanoat auf eine verhältnismässig zeitraubende Weise wie in der EP-B-0005830 beschrieben ist, hergestellt werden.

In der EP-A-0399094 wird ein verbessertes Verfahren zur Herstellung von Cefdinatriumsalz-hemiheptahydrat beschrieben,wonach 7-Amino-3-{[(2,5-dihydro-2-methyl-6-hydroxy-5-oxo-*as*-triazin-3-yl)thio]methyl)-3-cephem-4-carbonsäure in wäßriger Lösung und einem geeigneten Lösungsmittel in Anwesenheit eines Amins, ausgewählt aus einer Gruppe, bestehend aus Trimethylamin, Triethylamin, 1,4-Dimethyl-piperazin, N-ethylpiperidin, Pyridin und Dimethylaminopyridin, mit 2-(2-Aminothiazol-4-yl)-*syn*-methoxy-iminoessigsäure-2-benzothiazolyl-thioester umgesetzt wird. Die erhaltene wäßrige Lösung des entstandenen Salzes wird anschliessend, ohne Isolierung, mit einem geeigneten Salz einer Base, ausgewählt aus einer Gruppe bestehend aus Dicyclohexylamin, Diphenylamin, Diisopropylamin, N-terc.-Butylcyclohexylamin und N,N-Dibenzylethylendiamin, behandelt, und der erhaltene Niederschlag wird in einem.geeigneten Lösungsmittel mit Natrium-2-ethylhexanoat zur gewünschten Verbindung umgesetzt. Es wird eine Ausbeute von etwa 85% angegeben.

Bei diesem Verfahren wurde die Löslichkeit der schwerlöslichen Ausgangskomponente dadurch erhöht, dass deren Amin- bzw. Triethylaminsalz hergestellt wurde, was gleichzeitig auch den Schutz der Carboxygruppe zur Folge hatte. Allerdings ist es bei dieser Umsetzung ungünstig, dass einige sekundäre Amine und deren Salze, wie z.B. N,N-Dibenzylethylendiamin-diacetat, für die weitere Verwendung gesondert hergestellt werden müssen. Durch diese zeitraubende Herstellung der Zwischenprodukte (Umsetzungsreagentien) wird die ansonsten sehr hohe Gesamtausbeute herabgesetzt.

Der Erfindung liegt die Aufgabe zugrunde, Ceftriaxon-dinatriumsalz-hemiheptahydrat auf eine neue, verbesserte und technologisch leicht durchführbare Weise herzustellen, bei der man die gewünschte Substanz mit einer hohen Gesamtausbeute und einer hohen Reinheit erhält. Die Umsetzung der einzelnen Stufen soll in wäßrigem Medium bei etwa Raumtemperatur und innerhalb kurzer Zeit verlaufen.

Diese Aufgabe wird durch das in Patentanspruch 1 angegebene Verfahren sowie durch das neue Zwischenprodukt 7-Amino-3-{[(2,5-dihydro-6-hydroxy-2-methyl-5-oxo-as-triazin-3-yl)thio]-methyl}-3-cephem-4-carbonsäure-dinatriumsalz gelöst.

Erfindungsgemäß stellt man zuerst einfach durch quantitative Neutralisation der 7-Amino-3-{[(2,5-dihydro-6-hydroxy-2-methyl-5-oxo-*as*-triazin-3-yl)thio]methyl}-3-cephem-4-carbonsäure in Form der freien Säure mit einer wäßrigen Natriumhydrogencarbonatlösung das Dinatriumsalz dieser Säure der Formel II 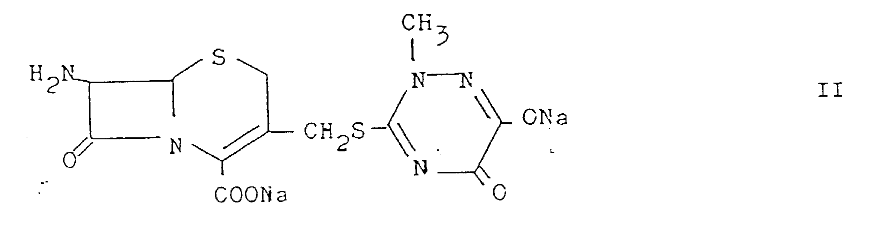 her, das eine neue und in der Literatur noch nicht beschriebene Verbindung ist. Das erhaltene Dinatriumsalz wird, ohne aus der wässrigen Lösung isoliert zu werden, anschliessend mit einem reaktiven 2-(2-Aminothiazol-4-yl)-2-*syn*-methoxy-iminoessigsäure-Derivat wie 2-(2-Aminothiazol-4-yl)-2-*syn*-methoxy-iminoessigsäure-2-benzothiazolyl-thioester in einem geeigneten inerten organischen Lösungsmittel wie Aceton, bei einer Temperatur zwischen 0 °C und 40 °C, vorzugsweise bei etwa Raumtemperatur (ca. 20-28°C) acyliert, und Ceftriaxondinatriumsalz-hemiheptahydrat wird anschließend in sehr reiner Form und hoher Gesamtausbeute isoliert.

Die Löslichkeit der Ausgangsverbindung, 7-Amino-3-{[(2,5-dihydro-6-hydroxy-2-methyl-5-oxo-*as*-triazin-3-yl)thio]methyl}-3-cephem-4-carbonsäure, wird auf einfache Weise durch quantitative Neutralisation mit Natriumhydrogencarbonat in wäßriger Lösung erzielt, wodurch die zeitraubende Herstellung des Ceftriaxondinatriumsalzes in der letzten Stufe und die Verwendung des teureren Natrium-2-ethylhexanoats vermieden wird.

Ausserdem entfällt der Schutz der Carboxygruppe in der Ausgangsverbindung, wie er in der oben angeführten Patentliteratur, z.B. in EP-A-0037380, beschrieben wird.

Als Acylierungsmittel kann das aus der Literatur bekannte und dann beschriebene reaktive 2-(2-Aminothiazol-4-yl)-2-*syn*-methoxyiminoessigsäure-Derivat, vorzugsweise ihr 2-Benzothiazolyl-thioester, eingesetzt werden, da es die vollständige Kontrolle der Geometrie der -C=N-*syn*-Konfiguration und dadurch eine hohe Ausbeute an syn-Isomeren der gewünschten Verbindung gewährleistet.

Der Reaktionsverlauf, die Identifizierung und der Gehalt an der gewünschten Verbindung wurden mittels Hochleistungs-Flüssigkeits-Chromatographie -Chromatographie (HPLC) auf schwach basischem Ionenaustauscher (Lichosorb-NH₂) mit einem Phosphatpuffer/ Acetonitril-Gemisch als mobile Phase und UV-Detektion bei 270 nm festgestellt. Die Reinheit der gewünschten Verbindung war hoch und mit der authentischen Probe vergleichbar.

Die Erfindung wird durch das folgende, in keiner Weise einschränkende Beispiel näher erläutert.

### BEISPIEL

200 mg (0,539 mMol) 7-Amino-3-{[(2,5-dihydro-6-hydroxy-2-methyl-5-oxo-*as*-triazin-3-yl)thio]methyl}-3-cephem-4-carbonsäure wurden in 4,5 ml Wasser suspendiert, mit 92 mg Natriumhydrogencarbonat (NaHCO₃) versetzt, und die Suspension wurde bis zur Klärung gerührt. Mit der erhaltenen Lösung wurde dann unter Rühren eine gesondert hergestellte Lösung von 230 mg (0,656 mMol) 2-(2-Aminothiazol-4-yl)-2-*syn*-methoxyiminoessigsäure-2-benzothiazolyl-thioester in 9 ml Aceton versetzt. Nach 4-stündigem Rühren bei Raumtemperatur wurde das Reaktionsgemisch filtriert. Das Filtrat wurde mit Aceton (7 ml) bis zur Trübheit versetzt, für 1 Stunde in den Gefrierschrank gestellt (wobei sich ein Niederschlag bildete), dann abermals mit 10 ml Aceton versetzt, und das erhaltene Reaktionsgemisch wurde über Nacht im Gefrierschrank stehengelassen. Danach wurde es mit weiteren 25 ml Aceton versetzt, erneut im Gefrierschrank abgekühlt, das ausgeschiedene Produkt wurde abgefiltert und unter Vakuum bei Raumtemperatur getrocknet. Es wurden 325 mg (91%) 7-{[2-(2-Aminothiazol-4-yl)-2-*syn*-methoxyimino]acetamido}-3-{[(2,5-di-hydro-6-hydroxy-2-methyl-5-oxo-*as*-triazin-3-yl)-thio]methyl}-3-cephem-4-carbon-säure-dinatriumsalz-hemiheptahydrat (Ceftriaxon-dinatriumsalz-hemiheptahydrat) von hoher Reinheit erhalten. Die analytischen Parameter waren mit denen der authentischen Probe identisch.
¹H NMR-Spektrum (DMSO) (δ, ppm)
6,76 5-Thiazolyl (1H, s)
3,83 -OCH₃ (3H, s)
3,42 -NCH₃ (3H, s)

### Herstellung und Isolierung von 7-Amino-3-{[2,5-dihydro-6-hydroxy-2-methyl-5-oxo-as-triazin-3-yl)thio]methyl}3-cephem-4-carbonsäure-dinatriumsalz

500 mg (1,35 mMol) 7-Amino-3-{[(2,5-dihydro-6-hydroxy-2-methyl-5-oxo-*as*-triazin-3-yl)thio]methyl}-3-cephem-4-carbonsäure wurden in 10 ml Wasser suspendiert, mit 230 mg Natriumhydrogencarbonat versetzt, dann mit 100 ml Aceton gefällt, und das erhaltene harzartige Produkt wurde über Nacht im Gefrierschrank stehengelassen, dann abgefiltert und in 30 ml Aceton suspendiert Nach einstündigem Rühren wurde das Produkt abgefiltert und in Vakuum bei 40 °C getrocknet. Es wurde 7-Amino-3-{[(2,5-dihydro-6-hydroxy-2-methyl-5-oxo-*as*-triazin-3-yl)thio]methyl}-3-cephem-4-carbonsäure-dinatriumsalz in der Form eines pulverigen, hellgelben Produktes, das bei einer Temperatur über 200 °C schmilzt, erhalten.

## Patentansprüche

1. Verfahren zur verstellung von 7-{[2-(2-Aminothiazol-4-yl)-2-*syn*-methoxyimino]acetamido}-3-{[(2,5-dihydro-6-hydroxy-2-methyl-5-oxo-*as*-triazin-3-yl)thio]methyl}-3-cephem-4-carbonsäure-dinatriumsalz-hemiheptahydrat (Ceftriaxondinatriumsalz-hemiheptahydrat) der Formel I 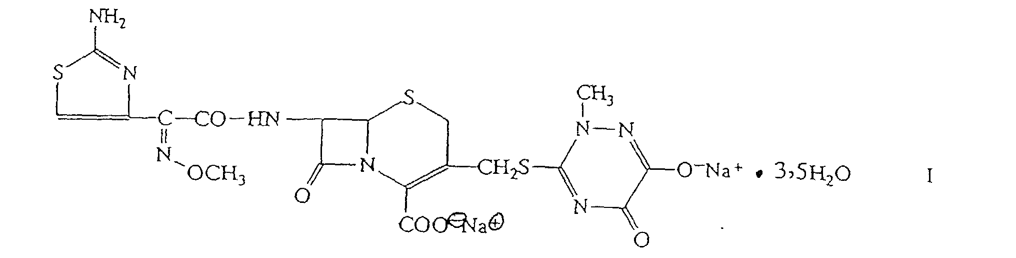 dadurch gekennzeichnet, dass man 7-Amino-3-{[(2,5-dihydro-6-hydroxy-2-methyl-5-oxo-*as*-triazin-3-yl)thio]methyl}-3-cephem-4-cärbonsäure mit Natriumhydrogencarbonat in wässrigem Medium zu 7-Amino-3-{[(2,5-dihydro-6-hydroxy-2-methyl-5-oxo-*as*-triazin-3-yl)thio]methyl}-3-cephem-4-carbonsäure-dinatriumsalz der Formel II 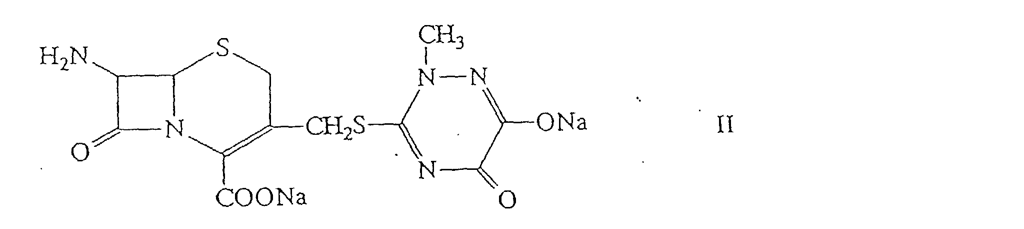 umsetzt und dann ohne Isolierung aus wässrigem Medium mit einem reaktiven 2-(2-Aminothiazol-4-yl)-2-*syn*-methoxyiminoessigsäure-Derivat der Formel III 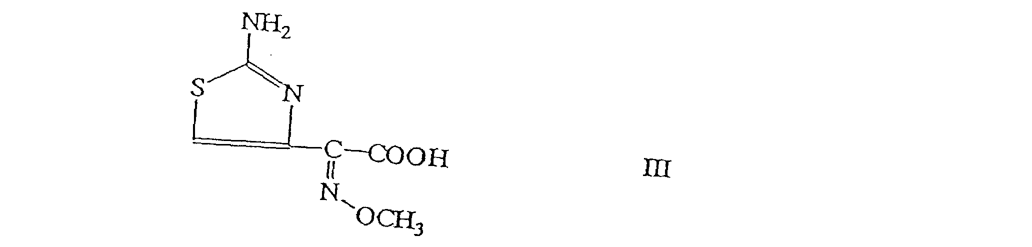 in einem geeigneten inerten organischen Lösungsmittel bei einer Temperatur zwischen 0°C und 40°C acyliert und dann das Ceftriaxon-dinatriumsalz-hemiheptahydrat in reiner Form isoliert.

2. Verfahren nach Anspruch 1, dadurch gekennzeichnet, daß man die Umsetzung bei Raumtemperatur durchführt.

3. Verfahren nach einem der Ansprüche 1 oder 2, dadurch gekennzeichnet, daß man als das reaktive Acylierungsderivat 2-(2-Aminothiazol-4-yl)-2-*syn*-methoxyiminoessigsäure-2-benzothiazolyl-thioester verwendet.

4. Verfahren nach einem oder mehreren der vorhergehenden Ansprüche 1 bis 3, dadurch gekennnzeichnet, daß man als inertes organisches Lösungsmittel Aceton verwendet.

5. Verfahren nach einem oder mehreren der vorhergehenden Ansprüche 1 bis 4, dadurch gekennzeichnet, daß man nach der Acylierungsstufe die Reaktionsmischung filtriert, zum Filtrat zusätzliches Aceton so lange zusetzt, bis Ceftriaxondinatriumsalz-hemiheptahydrat der Formel I in reiner Form erhalten wird, diese isoliert und trocknet.

6. 7-Amino-3-{[(2,5-dihydro-6-hydroxy-2-methyl-5-oxo-*as*-triazin-3-yl)-thio]methyl}-3-cephem-4-carbonsäure-dinatriumsalz.

## Claims

1. A process for the preparation of 7-{[2-(2-aminothiazol-4-yl)-2-*syn*-methoxyimino]acetamido}-3-{[(2,5-dihydro-6-hydroxy-2-methyl-5-oxo-*as*-triazin-3-yl)thio]methyl}-3-cephem-4-carboxylic acid disodium salt hemiheptahydrate (ceftriaxon disodium salt hemiheptahydrate) of formula I 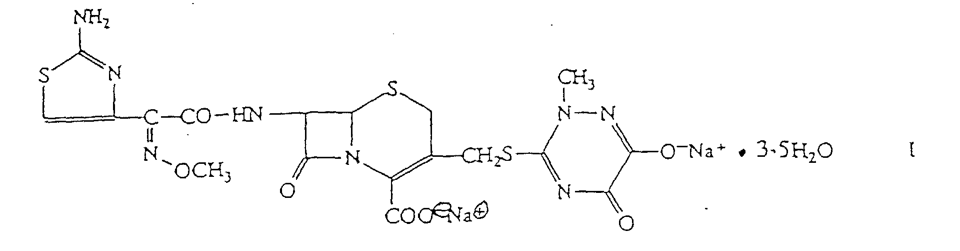 characterized in that 7-amino-3-{[(2,5-dihydro-6-hydroxy-2-methyl-5-oxo-*as*-triazin-3-yl)-thio]-methyl}-3-cephem-4-carboxylic acid is reacted with sodium hydrogen carbonate in an aqueous medium to 7-amino-3-{[(2,5-dihydro-6-hydroxy-2-methyl-5-oxo-*as*-triazin-3-yl)thio]methyl}-3-cephem-4-carboxylic acid disodium salt of formula II 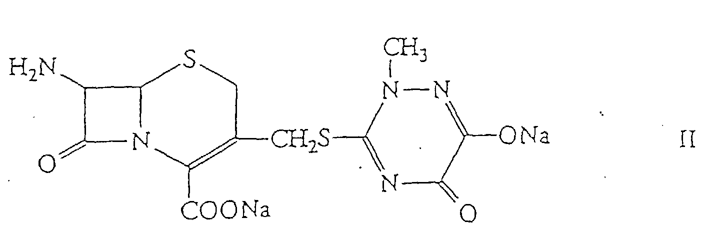 and then, without isolation from an aqueous medium, it is acylated with a reactive 2-(2-aminothiazol-4-yl)-2-*syn*-methoxyimino-acetic acid derivative of formula III 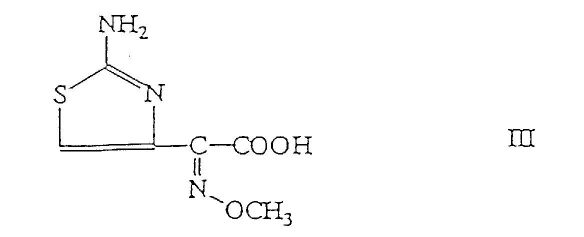 in a suitable inert organic solvent at a temperature between 0 °C and 40 °C and then ceftriaxon disodium salt hemiheptahydrate is isolated in a pure form.

2. A process according to claim 1, characterized in that the reaction is carried out at room temperature.

3. A process according to any of claims 1 or 2, characterized in that 2-(2-aminothiazol-4-yl)-2-*syn*-metoxyimino acetic acid-2-benzothiazolyl-thioester is used as the reactive acylating derivative.

4. A process according to any of previous claims 1 to 3, characterized in that acetone is used as the inert organic solvent.

5. A process according to any of previous claims 1 to 4, characterized in that after the acylating step the reaction mixture is filtered, additional acetone is added to the filtrate until ceftriaxon disodium salt hemiheptahydrate of the formula I is obtained in a pure form, the latter is isolated and dried.

6. 7-amino-3-{[(2,5-dihydro-6-hydroxy-2-methyl-5-oxo-*as*-triazin-3-yl)thio]methyl}-3-cephem-4-carboxylic acid disodium salt.

## Revendications

1. Procédé de préparation d'un sel disodique hémiheptahydraté d'acide 7-{[2-(2-aminothiazole-4-yl)-2-*syn*-méthoxyimino]acétamido}-3-{[(2,5-dihydro-6-hydroxy-2-méthyl-5-oxo-*as*-triazine-3-yl)thio]-méthyl}-3-céphème-4-carboxylique (sel disodique hémiheptahydraté de Céftriaxone) représenté par la formule I 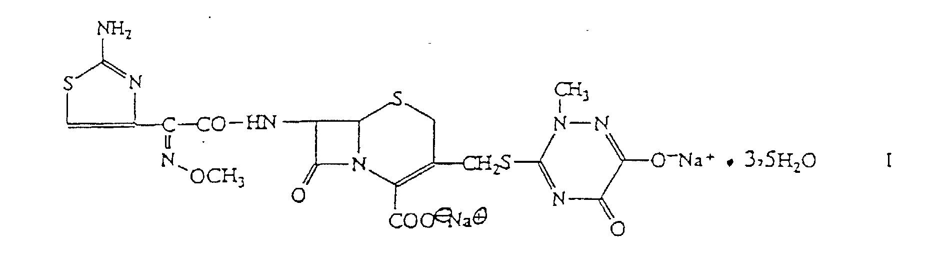 caractérisé en ce que l'on fait réagir l'acide 7-amino-3-{[2,5-dihydro-6-hydroxy-2-méthyl-5-oxo-*as*-triazine-3-yl)thio] méthyl}-3-céphème-4-carboxylique avec de l'hydrogéno-carbonate de sodium dans un milieu aqueux, en obtenant un sel disodique d'acide 7-amino-3-{[2,5-dihydro-6-hydroxy-2-méthyl-5-oxo-*as*-triazine-3-yl)thio]méthyl}-3-céphème-4-carboxylique représenté par la formule II 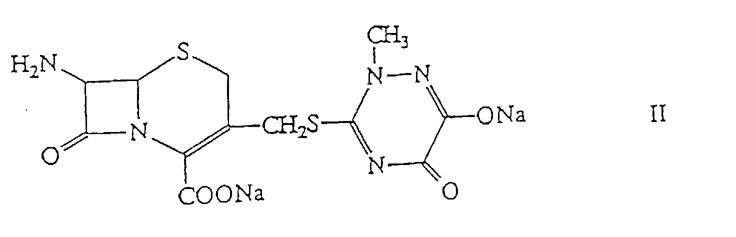 et ensuite, sans l'isoler du milieu aqueux, on l'acyle par un dérivé réactif d'acide 2-(2-aminothiazole-4-yl)-2-*syn*-méthoxyiminoacétique représenté par la formule III 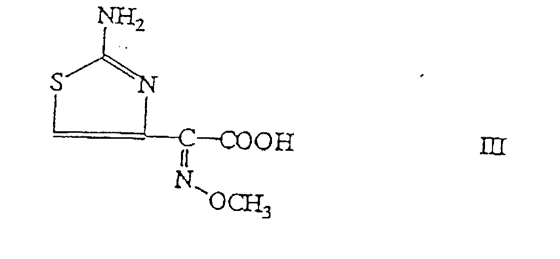 dans un solvant organique inerte approprié à une température comprise entre 0°C et 40°C, et enfin, on isole le sel disodique hémiheptahydraté de Céftriaxone à l'état pur.

2. Procédé selon la revendication 1, caractérisé en ce que la réaction s'effectue à une température ambiante.

3. Procédé selon une des revendications 1 ou 2, caractérisé en ce que l'on utilise, comme dérivé actif d'acylation, un benzothiazolyl-2 thioester d'acide 2-(2-aminothiazole-4-yl)-2-*syn*-méthoxyiminoacétique.

4. Procédé selon l'une ou plusieurs des revendications 1 à 3, caractérisé en ce que l'on utilise l'acétone comme solvant organique inerte.

5. Procédé selon l'une ou plusieurs des revendications 1 à 4, caractérisé en ce que, après l'acylation, on filtre le mélange réactionnel, on ajoute de l'acétone supplémentaire au filtrat jusqu'à l'obtention du sel disodique hémiheptahydraté de Céftriaxone de la formule I à l'état pur, on l'isole et on le sèche.

6. Sel disodique hémiheptahydraté d'acide 7-amino-3-{[(2,5-dihydro-6-hydroxy-2-méthyl-5-oxo-*as*-triazine-3-yl)thio]-méthyl}-3-céphème-4-carboxylique.
